# EUROPEAN PATENT SPECIFICATION

(11) **EP 2 661 962 B1**
(45) Date of publication and mention of the grant of the patent: **18.07.2018**
(21) Application number: 13165891.6
(22) Date of filing: 30.04.2013
(51) Int. Cl.: A61L 2/08, A61L 2/10, A01M 1/04, A01M 1/22

(54) **Insect pest disinfestation lighting device**
Beleuchtungsvorrichtung zur Entwesung von Insektenplagen
Dispositif d'éclairage pour la désinfestation des insectes nuisibles

(30) Priority: 11.05.2012 JP 2012110053
(43) Date of publication of application: 13.11.2013
(73) Proprietor: Panasonic Intellectual Property Management Co., Ltd., Osaka-shi, Osaka 540-6207 (JP)
(72) Inventor: Aoki, Shinichi, Chuo-ku, Osaka 540-6207 (JP); Yamada, Makoto, Chuo-ku, Osaka 540-6207 (JP); Ishiwata, Masaki, Chuo-ku, Osaka 540-6207 (JP)
(74) Representative: Appelt, Christian W.

(56) References cited:
- EP-A1- 0 007 459
- US-A- 4 566 220
- DATABASE WPI Week 201103 Thomson Scientific, London, GB; AN 2010-Q28907 XP002711519, -& CN 201 624 077 U (SHENZHEN JIUZHOU ELECTRIC GROUP CO LTD) 10 November 2010 (2010-11-10)

## Description

The present invention relates to an insect pest disinfestation lighting device.

Insect pests, such as spider mites, feed on plants and are thus troublesome. Japanese Laid-Open Patent Publication No. 2011-72200 describes the use of light to attract phytoseiidae, which are predators of spider mites, to disinfest spider mites.

The insect pest disinfestation process described above involves the attraction of phytoseiidae. It is thus desirable that a lighting device be developed to disinfest spider mites without attracting phytoseiidae.

Document CN 201624077 U discloses a device for avoidance of insects comprising an LED located behind a transparent cover. The insects are attracted by the LED light and hit a transparent cover and may ultimately be killed.

Document EP 0 007 459 A1 discloses a method for treatment for avoidance of plant diseases, especially for avoidance of microorganisms, bacteria and viruses, using a UV light for treatment of the plants.

It is an object of the present invention to provide an insect pest disinfestation lighting device that disinfests spider mites. The object is solved by an insect pest disinfestation lighting device according to claim 1; claim 2 to 6 relate to specifically advantageous realization of such an insect pest disinfestation lighting device.

Other embodiments and advantages of the present invention will become apparent from the following description, taken in conjunction with the accompanying drawings, illustrating by way of example the principles of the invention.

The invention, together with objects and advantages thereof, may best be understood by reference to the following description of the presently preferred embodiments together with the accompanying drawings in which:
Fig. 1 is a schematic diagram illustrating one embodiment of an insect pest disinfestation lighting system including an insect pest disinfestation lighting device;
Fig. 2 is a graph schematically illustrating the spectral properties of a first light source;
Fig. 3 is a graph schematically illustrating the spectral properties of a second light source;
Fig. 4 is a table illustrating test results for conditions A to E and comparative examples 1 to 4; and
Fig. 5 is a schematic block diagram illustrating the electrical configuration of the insect pest disinfestation lighting system.

One embodiment of an insect pest disinfestation lighting system will now be described with reference to the drawings.

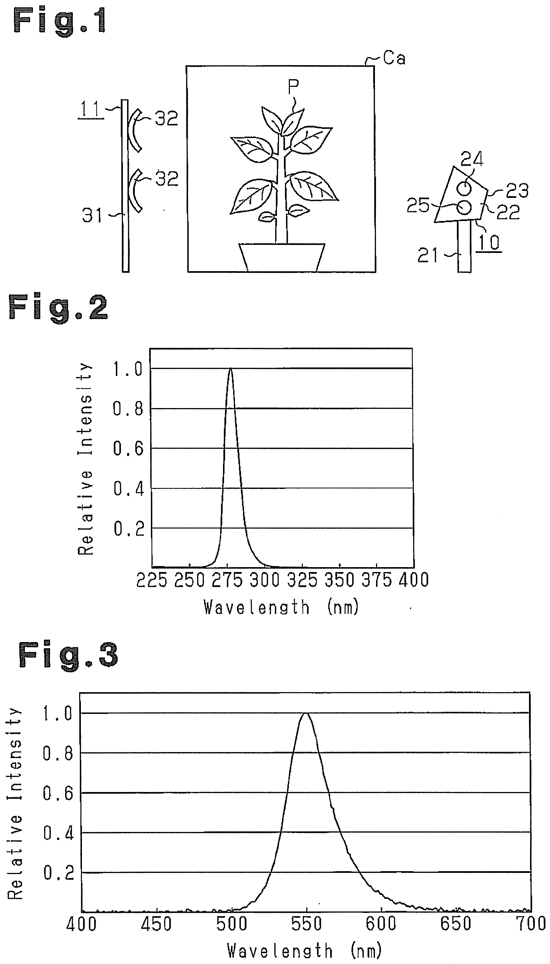

Referring to Fig. 1, the insect pest disinfestation lighting system includes an insect pest disinfestation lighting device 10 and a light reflector 11. A plant P is arranged between the insect pest disinfestation lighting device 10 and the light reflector 11.

The insect pest disinfestation lighting device 10 includes a lighting body 22, which is coupled to a distal end of a cylindrical pole 21. The lighting body 22 includes a first light source 24 and a second light source 25, which are arranged in a box-shaped housing 23. The first and second light sources 24 and 25 emit light of different spectral distributions. For example, LEDs may be used as the light sources 24 and 25.

The first light source 24 outputs light (first light) having a wavelength from 260 to 305 nm (UV light). In this embodiment, referring to Fig. 2, the first light source 24 outputs light having a peak wavelength of 280 nm.

The second light source 25 outputs light (second light) having a wavelength from 490 to 565 nm (green light). In this embodiment, referring to Fig. 3, the second light source 25 outputs light having a peak wavelength of 550 nm. The second light output from the second light source 25 functions to attract spider mites.

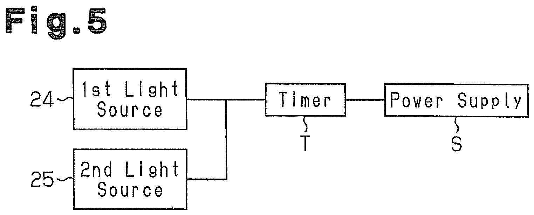

As illustrated in Fig. 5, the first and second light sources 24 and 25 are electrically connected to a timer T. The timer T determines whether or not to supply power from a power supply S to the first and second light sources 24 and 25. Thus, the first and second light sources 24 and 25 may be supplied with power from the power supply S at different set times. For example, the lighting device 10 illuminates the plant P with light from the first and second light sources 24 and 25 at predetermined time periods between sunset and sunrise.

As illustrated in Fig. 1, the light reflector 11 includes a plurality of (two illustrated in Fig. 1) reflection plates 32 arranged on a cylindrical support 31. The reflection plates 32 reflect light from the first light source 24 and the second light source 25. For example, aluminum material manufactured by Alanod may be used as a reflective material that reflects UV light. Further, for example, Miro (registered trademark), which is manufactured by Alanod, may be used as a reflective material that reflects green light. Additionally, aluminum reflection plates, silver reflection plates, and melamine reflection plates may be used as reflection plates for green light.

The insect pest disinfestation lighting device 10 activates the light sources 24 and 25 when supplied with power from the power supply S. When at least one of the light sources 24 and 25 is activated, the reflection plates 32 of the light reflector 11 reflect the light that did not illuminate the plant P. The reflected light illuminates the plant P.

### [Test]

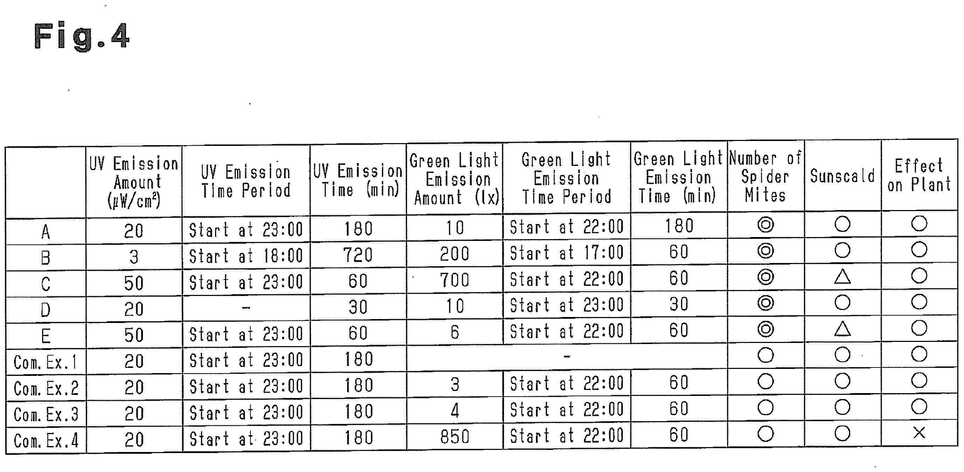

The inventors of the present invention conducted a test to evaluate spider mite disinfestation effects resulting from different activation time lengths and activation time periods of the light sources 24 and 25 in the insect pest disinfestation lighting device 10. Referring to Fig. 4, the test was conducted with the light sources 24 and 25 of the insect pest disinfestation lighting device 10 under conditions A to E and on comparative examples 1 to 4.

### [Condition A]

The first light source 24 emitted light with a UV emission amount of 20 µW/cm² for 180 minutes starting at 23:00. The second light source 25 emitted green light with an emission amount of 10 lx for 180 minutes starting at 22:00.

### [Condition B]

The first light source 24 emitted light with a UV emission amount of 3 µW/cm² for 720 minutes starting at 18:00. The second light source 25 emitted green light with an emission amount of 200 lx for 60 minutes starting at 17:00.

### [Condition C]

The first light source 24 emitted light with a UV emission amount of 50 µW/cm² for 60 minutes starting at 23:00. The second light source 25 emitted green light with an emission amount of 700 lx for 60 minutes starting at 22:00.

### [Condition D]

The first light source 24 emitted light with a UV emission amount of 20 µW/cm² for 30 minutes from when the second light source 25 completed the emission of light. The second light source 25 emitted green light with an emission amount of 10 lx for 30 minutes starting at 23:00.

### [Condition E]

The first light source 24 emitted light with a UV emission amount of 50 µW/cm² for 60 minutes starting at 23:00. The second light source 25 emitted green light with an emission amount of 6 lx for 60 minutes starting at 22:00.

### [Comparative Example 1]

The first light source 24 emitted UV light under condition A. The second light source 25 was not used.

### [Comparative Example 2]

The first light source 24 emitted UV light under condition A. The second light source 25 emitted green light with an emission amount of 3 lx for 60 minutes starting at 22:00.

### [Comparative Example 3]

The first light source 24 emitted UV light under condition A. The second light source 25 emitted green light with an emission amount of 4 lx for 60 minutes starting at 22:00.

### [Comparative Example 4]

The first light source 24 emitted UV light under condition A. The second light source 25 emitted green light with an emission amount of 850 lx for 60 minutes starting at 22:00.

### [Test Procedures]

Prior to the test, the plant P (e.g., cucumber) was placed in a transparent container Ca, the dimensions of which was approximately 1 m × 1 m × 1.5 m, and cultivated in the usual manner. Five spider mites were arranged on lower leaves of the plant P five to seven days after starting the cultivation of the plant P. The insect pest disinfestation lighting device 10 was activated three days after the arrangement of the spider mites under the above conditions. The test was conducted under the same conditions on three plants P in three transparent containers Ca to check the effects using the average observation value of the three plants P. The effects were checked based on three items, namely, the number of spider mites, sunscald, and effect on the plant P other than sunscald. The results are illustrated in Fig. 4. The number of spider mites was visually counted using a magnifying lens. In the table, with regard to spider mites, a single circle indicates that the presence of spider mites was easily recognized, and a double-circle indicates that the presence of spider mites was hardly recognized. With regard to sunscald, a triangle indicates that sunscald was recognized on one or two leaves of a single plant P, and a circle indicates that sunscald was hardly recognized. As the effect on the plant P other than sunscald, the generation of turion was visually checked. A cross indicates that there was an effect on the plant P, and a circle indicates that there was no effect on the plant P.

### [Evaluation Results of Conditions A to E]

As illustrated in Fig. 4, in the structure of comparative example 1, the presence of spider mites was easily recognized. Under conditions A to E, the presence of spider mites was hardly recognized. From these results, it may be understood that the emission of light from the second light source 25 in addition to the first light source 24 disinfests spider mites. Further, it may be understood from the results of conditions A to E that the illumination of the plant P with the light of 260 to 305 nm in the range of 3 to 50 µW/cm² is preferable.

In addition, it may be understood from the results of conditions A to E that the illumination of the plant P with light having the wavelength of 490 to 565 nm be emitted in the range of 6 to 700 lx from the second light source 25. This is because a slight amount of spider mites were recognized when the emission amount of green light was less than 6 lx (3 and 4 lx) like in comparative examples 2 and 3. Further, for example, like in comparative example 4, when the emission amount of green light was greater than 700 lx (850 lx), a slight amount of spider mites and an effect on the plant P were recognized.

The advantages of the insect pest disinfestation lighting device 10 of the present embodiment will now be described.
(1) The insect pest disinfestation lighting device 10 includes the first light source 24, which outputs the first light in the wavelength of 260 to 305 nm, and the second light source 25, which outputs second light in the wavelength of 490 to 565 nm, and illuminates the plant P with light from the light sources 24 and 25. This structure attracts spider mites with the light from the second light source 25 and then impairs and disinfests spider mites with the light from the first light source 24.
(2) Spider mites are likely to be produced on the rear sides of leaves. The light sources 24 and 25 illuminate the rear sides of the leaves of the plant P with light. This effectively disinfests spider mites.
(3) The first light source 24 illuminates the plant P with light of 260 to 305 nm in the range of 3 to 50 µW/cm². Such a structure decreases sunscald and other effects on the plant P while disinfesting spider mites.
(4) The second light source 25 illuminates the plant P with light of 490 to 565 nm in the range of 6 to 700 lx. Such a structure decreases sunscald and other effects on the plant P while disinfesting spider mites.
(5) The insect pest disinfestation lighting device 10 outputs light having a wavelength of 490 to 565 nm from the second light source 25 and then outputs light having a wavelength of 260 to 305 nm from the first light source 24. This structure attracts spider mites with light in the wavelength of 490 to 565 nm and then impairs spider mites with light in the wavelength of 260 to 305 nm.
(6) The light sources 24 and 25 illuminate the plant P with light during predetermined time periods from sunset to sunrise. The second light source 25 emits green light during the nighttime to improve the spider mite attraction efficiency during the nighttime when there is not much sunlight. During the nighttime, there is no sunlight, and spider mites respond to even a small amount of light. This lengthens the moving distance of a spider mite, especially, an imago, responding to light from the light sources 24 and 25 (especially, the light source 25). Thus, the time is increased during which the spider mite is illuminated with UV light from the first light source 24, and the emission of UV light from the first light source 24 during the nighttime disinfests spider mites in a further preferable manner. Further, there may be no workers during the nighttime. This allows for reduction in the UV light emitted toward workers from the light source 24.

It should be apparent to those skilled in the art that the present invention may be embodied in many other specific forms without departing from the spirit or scope of the invention. Particularly, it should be understood that the present invention may be embodied in the following forms.

In the above embodiment, the first light source 24 and the second light source 25 are accommodated in the same housing 23. Instead, for example, the first light source 24 and the second light source 25 may be accommodated in different housings.

In the above embodiment, the first light source 24 and the second light source 25 start emitting light at different times but may start emitting light at the same time. Further, in the above embodiment, the first light source 24 emits light after the second light source 25. Instead, the second light source 25 may emit light after the first light source 24.

In the above embodiment, the first light source 24 has the spectral properties illustrated in Fig. 2, and the second light source 25 has the spectral properties illustrated in Fig. 3. However, the light sources 24 and 25 are not limited to the spectral properties of the above embodiment. Further, the light sources 24 and 25 are not limited to LEDs and may be formed by combining a fluorescent lamp or another light source with a filter. As long as light in the wavelength band of the first light source 24 and light in the wavelength band of the second light source 25 may be output, the types of the light sources 24 and 25 are not particularly limited.

The present examples and embodiments are to be considered as illustrative and not restrictive, and the invention is not to be limited to the details given herein, but may be modified within the scope of the appended claims.

## Claims

1. An insect pest disinfestation lighting device (10) comprising a light source (24, 25), the insect pest disinfestation lighting device (10) comprising a first light source (24) configured to illuminate a plant (P) with first light having a wavelength of 260 to 305 nm,
a second light source (25) configured to illuminate a plant (P) having a wavelength of 490 to 565 nm, and
a timer (T) electrically connected to the first and second light sources (24, 25) configured to control a power supply (S) to the first and second light sources (24, 25) at different set times.

2. The insect pest disinfestation lighting device (10) according to claim 1, wherein the first light source (24) and the second light source (25) are arranged to illuminate a rear side of a leaf of the plant (P) with the first light and the second light.

3. The insect pest disinfestation lighting device (10) according to claim 1 or 2, wherein the first light source (24) is configured to illuminate the plant (P) with the first light having the wavelength of 260 to 305 nm in a range of 3 to 50 µW/cm².

4. The insect pest disinfestation lighting device (10) according to any one of claims 1 to 3, wherein the second light source (25) is configured to illuminate the plant (P) with the second light having the wavelength of 490 to 565 nm in a range of 6 to 700 lx.

5. The insect pest disinfestation lighting device (10) according to any one of claims 1 to 4, wherein the timer (T) is configured to control a power supply (S) such that the first light source (24) outputs the first light having the wavelength of 260 to 305 nm after the second light source (25) outputs the second light having the wavelength of 490 to 565 nm.

6. The insect pest disinfestation lighting device (10) according to any one of claims 1 to 5, wherein the timer (T) is configured to control the power supply (S) such that the first light source (24) and the second light source (25) illuminate the plant (P) with the first light and the second light during a predetermined time period between sunset and sunrise.

## Patentansprüche

1. Beleuchtungsvorrichtung (10) zur Ungezieferbekämpfung von Insektenplagen, mit einer Lichtquelle (24, 25), wobei die Beleuchtungsvorrichtung (10) zur Ungezieferbekämpfung von Insektenplagen aufweist:
eine erste Lichtquelle (24), die dazu eingerichtet ist, eine Pflanze (P) mit einem ersten Licht zu beleuchten, das eine Wellenlänge von 260 bis 305 nm aufweist,
eine zweite Lichtquelle (25), die dazu eingerichtet ist, eine Pflanze (P) mit einer Wellenlänge von 490 bis 565 nm zu beleuchten, und
einen Zeitgeber (T), der mit der ersten und der zweiten Lichtquelle (24, 25) verbunden ist und dazu eingerichtet ist, eine Stromzufuhr (S) zu der ersten und zu zweiten Lichtquelle (24, 25) zu unterschiedlichen eingestellten Zeiten zu steuern.

2. Beleuchtungsvorrichtung (10) zur Ungezieferbekämpfung von Insektenplagen nach Anspruch 1, wobei die erste Lichtquelle (24) und die zweite Lichtquelle (25) dazu eingerichtet sind, eine Rückseite eines Blattes der Pflanze (P) mit dem ersten Licht und dem zweiten Licht zu beleuchten.

3. Beleuchtungsvorrichtung (10) zur Ungezieferbekämpfung von Insektenplagen nach Anspruch 1 oder 2, wobei die erste Lichtquelle (24) dazu eingerichtet ist, die Pflanze (P) mit dem ersten Licht, das die Wellenlänge von 260 bis 305 nm aufweist, in einem Bereich von 3 bis 50 µW/cm² zu beleuchten.

4. Beleuchtungsvorrichtung (10) zur Ungezieferbekämpfung von Insektenplagen nach einem beliebigen der Ansprüche 1 bis 3, wobei die zweite Lichtquelle (25) dazu eingerichtet ist, die Pflanze (P) mit dem zweiten Licht, das die Wellenlänge von 490 bis 565 nm aufweist, in einem Bereich von 6 bis 700 lx zu beleuchten.

5. Beleuchtungsvorrichtung (10) zur Ungezieferbekämpfung von Insektenplagen nach einem beliebigen der Ansprüche 1 bis 4, wobei der Zeitgeber (T) dazu eingerichtet ist, eine Stromzufuhr (S) so zu steuern, dass die erste Lichtquelle (24) das erste Licht mit der Wellenlänge von 260 bis 305 nm ausgibt, nachdem die zweite Lichtquelle (25) das zweite Licht mit der Wellenlänge von 490 bis 565 nm ausgibt.

6. Beleuchtungsvorrichtung (10) zur Ungezieferbekämpfung von Insektenplagen nach einem beliebigen der Ansprüche 1 bis 5, wobei der Zeitgeber (T) dazu eingerichtet ist, die Stromzufuhr (S) so zu steuern, dass die erste Lichtquelle (24) und die zweite Lichtquelle (25) die Pflanze (P) während einer vorbestimmten Zeitspanne zwischen Sonnenuntergang und Sonnenaufgang mit dem ersten Licht und dem zweiten Licht beleuchten.

## Revendications

1. Dispositif d'éclairage pour la désinfestation des insectes nuisibles (10) comprenant une source de lumière (24, 25), le dispositif d'éclairage pour la désinfestation des insectes nuisibles (10) comprenant
une première source de lumière (24) configurée pour éclairer une plante (P) avec une première lumière ayant une longueur d'onde de 260 à 305 nm,
une seconde source de lumière (25) configurée pour éclairer une plante (P) ayant une longueur d'onde de 490 à 565 nm, et
un minuteur (T) électriquement relié à la première et à la seconde sources de lumière (24, 25) configuré pour contrôler une alimentation électrique (S) de la première et de la seconde sources de lumière (24, 25) à différents moments définis.

2. Dispositif d'éclairage pour la désinfestation des insectes nuisibles (10) selon la revendication 1,
dans lequel la première source de lumière (24) et la seconde source de lumière (25) sont prévues pour éclairer un côté arrière d'une feuille de la plante (P) avec la première lumière et la seconde lumière.

3. Dispositif d'éclairage pour la désinfestation des insectes nuisibles (10) selon la revendication 1 ou 2,
dans lequel la première source de lumière (24) est configurée pour éclairer la plante (P) avec la première lumière ayant la longueur d'onde de 260 à 305 nm sur une plage de 3 à 50 µW/cm².

4. Dispositif d'éclairage pour la désinfestation des insectes nuisibles (10) selon l'une quelconque des revendications 1 à 3, dans lequel la seconde source de lumière (25) est configurée pour éclairer la plante (P) avec la seconde lumière ayant la longueur d'onde de 490 à 565 nm sur une plage de 6 à 700 lx.

5. Dispositif d'éclairage pour la désinfestation des insectes nuisibles (10) selon l'une quelconque des revendications 1 à 4, dans lequel le minuteur (T) est configuré pour contrôler une alimentation électrique (S) de sorte que la première source de lumière (24) délivre la première lumière ayant la longueur d'onde de 260 à 305 nm après que la seconde source de lumière (25) a délivré la seconde lumière ayant la longueur d'onde de 490 à 565 nm.

6. Dispositif d'éclairage pour la désinfestation des insectes nuisibles (10) selon l'une quelconque des revendications 1 à 5, dans lequel le minuteur (T) est configuré pour contrôler l'alimentation électrique (S) de sorte que la première source de lumière (24) et la seconde source de lumière (25) éclairent la plante (P) avec la première lumière et la seconde lumière pendant une durée prédéterminée comprise entre le crépuscule et l'aurore.
